(19) Europäisches Patentamt
European Patent Office
Office européen des brevets

(11) Numéro de publication: **0 104 101**
**B1**

(12) **FASCICULE DE BREVET EUROPÉEN**

(45) Date de publication du fascicule du brevet: **25.02.87**

(21) Numéro de dépôt: **83401640.4**

(22) Date de dépôt: **16.08.83**

(51) Int. Cl.[4]: **C 08 F 216/34,** C 08 F 220/28, G 01 N 33/53 // (C08F216/34, 220:28), (C08F220/28, 220:34)

(54) **Réactif permettant un dosage de très haute sensibilité de l'antigène caractéristique du virus de l'hépatite B dans les liquides biologiques humains.**

(30) Priorité: **16.08.82 FR 8214170**

(43) Date de publication de la demande: **28.03.84 Bulletin 84/13**

(45) Mention de la délivrance du brevet: **25.02.87 Bulletin 87/9**

(84) Etats contractants désignés: **AT BE CH DE FR GB IT LI LU NL SE**

(56) Documents cité:
**FR-A-1 599 421**
**FR-A-2 258 406**
**FR-A-2 259 847**
**FR-A-2 289 533**

(73) Titulaire: **SANOFI, société anonyme, 40, Avenue George V, F-75008 Paris (FR)**

(72) Inventeur: **Duheille, Jean Emile Louis, 22 rue des Magnolias, F-54130 Saint- Max (FR)**
Inventeur: **Pau, Bernard Claude, Les Allées du Bois Bt 638, rue d'Uppsala, La Paillade F-34100 Montpellier (FR)**
Inventeur: **Gros, Pierre Marcel, Les Fontaines 18 rue des Muriers, F-34100 Montpellier (FR)**

(74) Mandataire: **Gillard, Marie- Louise, Cabinet Beau de Loménie 55, Rue d'Amsterdam, F-75008 Paris (FR)**

**EP 0 104 101 B1**

*Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).*

LIBER, STOCKHOLM 1987

## Description

La présente invention concerne des réactifs utiles pour le diagnostic des hépatites virales de type B, un procédé pour la préparation de ces réactifs et un procédé pour utiliser ces réactifs en vue de réaliser le dosage quantitatif de très haute sensibilité de l'antigène caractéristique du virus de l'hépatite B dans le sérum humain.

Les hépatites virales sont des maladies infectieuses transmissibles très fréquentes, dont l'agent causal est un virus et qui se caractérisent habituellement par une nécrose et une inflammation hépatiques, fréquemment accompagnées d'ictère. Bien que ces maladies soient généralement bénignes, près de 1 % des cas sont mortels et environ 5 % des cas conduisent à des formes prolongées ou chroniques. On distingue les hépatites de type A dont le virus est principalement transmis par voie orale (nourriture, boisson), les hépatites de type B dont le virus est le plus souvent transmis par voie parentérale et enfin d'autres hépatites (non A - non B). En ce qui concerne les hépatites de type B les sujets les plus susceptibles de contracter la maladie sont ceux qui sont soumis à des transfusions sanguines ou des hémodialyses, le personnel médical et paramédical, le personnel des centres de transfusion sanguine. Le virus de l'hépatite B est de ce fait recherché en dépistage systématique chez tous les donneurs de sang car il est connu que le virus peut persister longtemps dans le sang de sujets apparemment sains.

La corrélation que l'on observe entre l'augmentation de sensibilité des techniques de dépistage et la réduction de fréquence des hépatites post-transfusionnelles plaide très fortement en faveur de la disponibilité de réactifs et de techniques de dosage de haute sensibilité aisées à mettre en oeuvre de réponse rapide et de coût peu élevé.

Il faut noter en outre la corrélation existant, notamment chez certaines populations africaines entre la présence dans le sang de virus de l'hépatite B et l'apparition de cancers primitifs du foie. Cette observation plaide également en faveur de possibilités de diagostic de masse chez ces populations.

L'antigène de surface du virus de l'hépatite B, dit antigène HB ou antigène Australia, est généralement reconnu comme le marqueur le plus important du virus et c'est cet antigène que l'on cherche à doser dans le sang. Les méthodes actuellement disponibles pour atteindre la sensibilité requise -le seuil de détection devant être inférieur à 1 nanogramme par millitre de sérum- sont toutes de nature radioimmunologique et présentent notamment les inconvénients suivants liés à la manipulation de substances radioactives : appareillages spécialisés et coûteux, problèmes de contamination possible (préjudiciables à la qualité du résultat et à la sécurité du personnel), obligation de disposer de personnel très qualifié et controlé sur le plan médical et radiotoxicologique, contraintes imposées par la loi pour l'aménagement des locaux et la sécurité, durée de validité limitée des réactifs ...

Les réactifs et procédés objets de la présente invention sont d'une nature radicalement différente, n'impliquant aucune manipulation de substances radioactives par l'utilisateur et évitant donc tous les inconvénients cités ci-dessus. Le principe retenu pour ces dosages est celui de la néphélémétrie à support microparticulaire de très haute sensibilité.

La néphélémétrie est une méthode physique très connue en chimie analytique. Lorsque le même principe est appliqué à la quantification de réactions antigène/anticorps, elle prend alors le nom d'immunonéphélémétrie. Elle a été principalement employée pour le dosage spécifique des protéines du sérum par la mesure de l'intensité de la lumière diffusée par les complexes immuns insolubles formés entre chacune de ces protéines et un immun-sérum correspondant. L'amélioration des appareillages, notamment par l'emploi de sources lumineuses à laser a permis d'atteindre des sensibilités de l'ordre du microgramme de protéine à doser par ml de liquide biologique.

Les appareils de ce type sont maintenant largement diffusés dans les laboratoires, mais la sensibilité ainsi atteinte est encore très insuffisante pour le dosage de l'antigène $HB_S$.

Dès 1976 BONNEFOY et GRANGE (C.R Acad. Sc. Paris D 283, 1976, 115-118) ont proposé d'améliorer les performances de l'immunoné phélémétrie par l'emploi de microsphères de polystyrène portant l'un des composants de la réaction antigène/anticorps à étudier. Cette modification introduit le principe de l'emploi des supports microparticulaires en immunonéphélémétrie.

Des travaux analogues ont fait l'objet de publications (voir notamment J. Immunol. Methods 18 1977 214-224; ibid 33 1980 159-173; Immunochemistry 13, 1976, 955-962 et 963-966; Molec. Immunol. 17, 1980, 81-92) et de brevets (notamment brevet déposé en France au nom de l'INSERM (inventeur G. A. QUASH) le 06/03/79). Ces techniques emploient de préference des microsphères de polystyrène sur lesquelles est fixé (par adsorption ou par covalence) le composant convenable de la réaction antigène/anticorps à étudier.

Les performances ont encore été améliorées par l'emploi de supports microparticulaires à caractère hydrophile par opposition aux précédents qui étaient hydrophobes et présentaient une instabilité par auto-agglutination et sédimentation lente et des risques liés notamment à des adsorptions non spécifiques. Ces nouveaux supports microparticulaires hydrophiles ont été décrits et brevetés notamment par REMBAUM et ses collaborateurs (REMBAUM et al. Macromolecules 9 (2), 1976 328-336; REMBAUM et al. J. Macromol. Sci. Chem. A 13 (5), 1979, 603-632; brevet français n° 2 258 406 du 17 janvier 1975; brevets des Etats-Unis d'Amérique n° 4 138 383 du 6 février 1979 et 4 206 094 du 3 janvier 1980) mais en vue d'utilisations biomédicales sans rapport avec l'immunonéphélémétrie, ni avec le dosage de l'antigène HB . Les supports du même type ont aussi été utilisés par DUHEILLE et ses collaborateurs (MONTAGNE et al. Groupe d'Etude Laser Behring, septembre 1979; MONTAGNE et al.,IVe Congrès International d'Immunologie - Paris 21-26 juillet 1980) pour le dosage immunonéphélémétrique des complexes immuns circulants. Toutefois, aucune technique de ce type n'a jamais

été appliquée au dosage de l'antigène $HB_s$.

Poursuivant ses études sur la formation du signal néphélémétrique en fonction de la taille du nombre et des caractéristiques des particules dispersantes la demanderesse a découvert le grand intêret de nouveaux supports microparticulaires qui se comportent comme indicateurs et amplificateurs remarquables du signal néphélémétrique. Ce sont ces supports et leur application au dosage de l'antigène HB qui font l'objet de la présente invention. Ces nouveaux supports ont les caractéristiques et avantages suivants:

Ce sont des microparticules sphériques, hydrophyles polyfonctionnelles, à charge électrique variable de façon controlée de taille très uniforme et parfaitement contrôlable dans la gamme de diamètres moyens allant d'environ 10 nanomètres à environ 10 micromètres.

De telles caractéristiques éminemment favorables au but poursuivi et qu'aucun des supports antérieurement décrits ne possède toutes ensemble, découlent de la nature chimique de ces particules qui résultent de la copolymérisation réalisée en milieu strictement aqueux d'au moins trois monomères acryliques hydrosolubles, en présence d'un agent de réticulation et d'un agent tensioactif, eux-mêmes également hydrosolubles.

Toutes les caractéristiques, géométriques (taille moyenne et dispersion des tailles autour de la moyenne), physiques (hydrophilie et densité de charges électriques superficielles) et chimiques (fonctions chimiques présentes en surface) de ces particules sont entièrement régies par le choix de la nature des constituants du milieu réactionnel de leurs proportions relatives et des conditions dans lesquelles a lieu la copolymérisation.

Les monomères acryliques utilisés sont choisis parmi les substances définies ci-après :

1) un aldéhyde acrylique de formule générale :

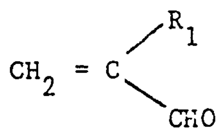

où R est l'hydrogène ou un groupe alkyle inférieur ayant de 1-4 atomes de carbone. Ce monomère représente au moins 5% en poids de l'ensemble des monomères, mais le plus souvent entre 40 et 60 %.

Il apporte des fonctions aldéhyde qui seront préservées lors de la polymérisation et seront présentes et accessibles à la surface des microsphères obtenues permettant ainsi la simplification et la diversification des procédures de couplage par covalence de protéines ou de glycoprotéines ou d'autres macromolécules ou molécules naturelles ou hémisynthétiques convenables à la surface des microphères.

2) un autre dérivé acrylique de formule générale

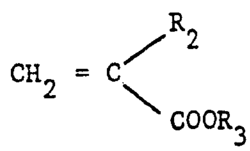

où $R_2$ est choisi parmi l'hydrogène et les groupes alkyle inférieur ayant de 1-4 atomes de carbone

$R_3$ est un groupe alkyle inférieur hydroxylé.

Ce monomère représente au moins 5 % en poids de l'ensemble des monomères et le plus souvent entre 40 et 70%. Il apporte des fonctions hydroxyles en grand nombre qui confèrent aux microsphères leur hydrophilie et un caractère parfaitement mouillable par les solvants aqueux, ce qui participe aussi à la stabilité du réactif.

3) moins de 15% en poids de l'ensemble des monomères d'au moins un dérivé acrylique choisi parmi les dérivés de formule générale:

a) 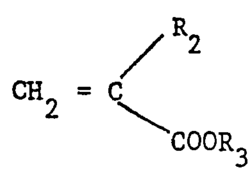

où $R_2$ est l'hydrogène ou un groupe alkyle inférieur ayant de 1-4 atomes de carbone

$R_3$ est l'hydrogène.

Ce monomère apporte aux microsphères des groupements carboxyliques dont le caractère anionique à pH physiologique est essentiel à la régulation de la charge électrique superficielle des microsphères et, par là, à la stabilité du réactif en suspension. Tout ou partie de ces groupements carboxyliques peuvent aussi être utilisés comme fonctions chimiques disponibles pour assurer le couplage covalent des molécules à fixer sur les microsphères, selon les techniques usuelles de la chimie.

et de formule générale:

b) 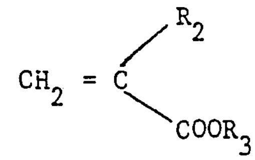

où $R_2$ a la même signification que précédemment et $COOR_3$ représente un groupe de structure

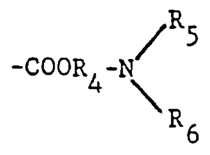

où $R_4$, $R_5$ et $R_6$ sont des groupes alkyle inférieurs identiques ou différents (de 1-4 atomes de carbone).

Ce monomère apporte aux microsphères des groupements amine tertiaire dont le caractère cationique à pH physiologique participe à la régulation de la charge électrique superficielle des microsphères et, par là, à la stabilité du réactif en supension.

Les produits selon l'invention seront donc obtenus à partir d'au moins trois monomères à savoir les monomères 1), 2) et au moins un monomère 3); c'est-à-dire que les quantités utilisées pour a) et b) ne peuvent pas être simultanément nulles.

Il est ainsi évident qu'un choix judicieux de la proportion des monomères appartenant aux classes a), b) et 2) définies ci-dessus règle exactement comme on le souhaite à la fois l'hydrophilie et la charge électrique nette des microsphères. Cette régulation est essentielle pour permettre au réactif d'atteindre la sensibilité maximale du dosage. En effet, la sensibilité du dosage sera maximale si la charge électrique des microsphères est réglée de telle sorte que les répulsions électrostatiques entre sphères soient juste assez fortes pour empêcher l'auto-aggrégation et néanmoins assez faibles pour permettre l'agglutination spécifique de deux sphères par la réaction antigène-anticorps minimale. Les mêmes conditions favorisent aussi l'accélération des réactions d'agglutination spécifiques, ce qui représente un élément très favorable pour la mise en oeuvre pratique du dosage.

Il faut enfin noter que l'absence de tout monomère hydrophobe permet d'obtenir un milieu réactionnel aqueux homogène, favorable à la plus grande régularité dans l'amorçage et le déroulement de la polymérisation, ce qui entraîne une excellente homogénéité de taille des sphères formées, quelle que soit la taille moyenne que l'on cherche à obtenir.

4 - En plus des monomères acryliques décrits ci-dessus, le milieu de polymérisation contient en outre un agent réticulant destiné à assurer les pontages entre chaînes linéaires de polymères acryliques et de ce fait la cohésion du réseau tridimensionnel de copolymère. Cet agent réticulant est caractérisé en ce qu'il est un diène non conjugué et notamment le N-N'-méthylène bis acrylamide, soluble dans l'eau et qu'il représente de 0,1 à 10 % en poids de l'ensemble des monomères et le plus souvent entre 0,5 et 5 %. La proportion choisie d'agent réticulant a également pour rôle de contrôler la porosité des microsphères obtenues.

5 - Enfin, le milieu de polymérisation contient un agent tensioactif qui peut être de type ionique -et notamment le dodécylsulfate de sodium- ou de type non ionique. Il est utilisé à une concentration comprise entre 0,01 % et 5 % en poids du milieu réactionnel total et son rôle principal est de régler les modalités de croissance des sphères pendant la polymérisation et de ce fait la taille finale des microsphères obtenues.

6 - Au cours de nos travaux, il a été reconnu que la taille finale des microsphères obtenues pouvait être le plus favorablement réglée en influant, d'une part, sur la concentration en agent tensioactif et d'autre part, sur la concentration finale en mélange total de manomères dans le milieu réactionnel de polymérisation. Toutes choses égales par ailleurs, le diamètre des microsphères obtenues est d'autant plus grand que la concentration de l'agent tensioactif est faible et que la concentration totale en monomères est élevée. La concentration totale en monomères dans le milieu réactionnel peut être choisie entre 4 et 16 % en poids.

7 - Le mélange des monomères choisis de l'agent réticulant et de l'agent tensioactif étant réalisé dans les conditions permettant d'obtenir des microsphères de caractéristiques voulues, ce milieu réactionnel est réparti dans des ampoules de verre borosilicaté de taille convenable qui sont ensuite scellées sous vide. Ces ampoules sont fixées sur un agitateur soit alternatif, soit rotatif et l'ensemble est placé dans la chambre d'irradiation d'une bombe au cobalt 60. L'irradiation qui provoque la réaction de polymérisation est réalisée à raison de 0,01 à 0,5 mégarad par heure et par $cm^3$ de milieu réactionnel pour une durée qui n'est jamais inférieure à 15 minutes et peut atteindre jusqu'à 10 heures.

Nos études ont également montré qu'à dose d'irradiation constante la taille des billes est plus grande si le flux est plus faible et la durée d'irradiation corrélativement plus élevée.

Après irradiation, les ampoules sont ouvertes et le milieu réactionnel dilué dans un égal volume d'une solution aqueuse réductrice (et notamment une solution à 1 g/l d'hydroquinone) afin, d'une part, d'arrêter les réactions de polymérisation et, d'autre part, de prévenir l'oxydation des fonctions aldéhyde présentes sur les

microsphères. Les préparations obtenues sont alors parfaitement stables et conservées à 4°C dans ces conditions jusqu'à l'emploi ultérieur. Les microsphères obtenues peuvent être examinées et contrôlées par microscopie électronique ce qui permet à la fois de déterminer la taille moyenne et la dispersion des tailles autour de cette moyenne. Dans les conditions employées, l'écart-type des tailles pour une préparation donnée est toujours inférieur à un dixième de la moyenne.

Pour pouvoir être utilisées en vue du dosage de l'antigène $HB_s$, les microsphères obtenues doivent encore être préalablement couplées chimiquement aux anticorps dirigés contre l'antigène $HB_s$ et qui provoqueront la réaction d'agglutination spécifique qui est la base de la formation du signal néphélémétrique. Les anticorps utilisables selon l'invention peuvent être :

- soit polyclonaux et isolés par les techniques connues à partir d'immun-sérums spécifiques obtenus chez l'animal après immunisation par l'antigène $HB_s$ purifié
- soit monoclonaux et purifiés par les méthodes connues après production à partir de cellules hybrides (hybridomes) résultant de la fusion de plasmocytes d'animaux immunisés contre l'antigène $HB_s$ et de cellules de myélome possédant les caractéristiques convenables.

Les anticorps utilisables peuvent aussi résulter de mélanges de plusieurs immunoglobulines spécifiques de l'antigène $HB_s$ afin de ne laisser aucun variant rare de l'antigène échapper au dosage.

Afin de coupler les anticorps anti-$HB_s$ aux microsphères choisies la suspension de microsphères est préalablement dialysée pour éliminer les excès de réactifs puis ajustée à une concentration connue et comprise entre 1 et 50 mg de particules par ml (concentration déterminée par poids sec) dans un milieu tamponné isotonique et additionnée d'une solution de l'anticorps dans le même solvant. Le couplage s'effectue spontanément en quelques heures à température ambiante par réaction des fonctions aminées de la protéine avec les groupes aldéhyde portés par les microsphères pour former des liaisons imines.

Après que la réaction a atteint le degré d'avancement requis (entre 1 et 30 heures), les groupements aldéhyde en excès sont bloqués par réaction avec un excès d'une amine primaire et de préférence d'une amine primaire hydroxylée telle que l'éthanolamine dont les groupements hydroxyle contribuent aussi à l'hydrophilie des particules ainsi revêtues. Cette étape est réalisée par incubation à température ambiante pendant à nouveau 1 à 10 heures.

Alternativement l'étape de blocage des groupes aldéhyde en excès peut être remplacée par une étape de réduction à l'aide d'un hydrure métallique et notamment le borohydrure de sodium. Ce traitement entraîne à la fois la réduction des fonctions imine en fonctions amine secondaire, ce qui stabilise la fixation de l'anticorps sur les microsphères et en même temps réduit les groupes aldéhyde en alcool ce qui comme précédemment favorise l'hydrophilie des particules.

La suspension des microsphères ainsi obtenues est ensuite purifiée par centrifugation en présence d'un gradient de densité convenable (par exemple un gradient de saccharose de 10 à 60 %). La couche contenant les particules est récupérée et finalement dialysée contre un tampon isotonique.

Une telle préparation d'immunosphères (microsphères portant un anticorps spécifique) peut être utilisée directement pour le dosage néphélémétrique de l'antigène $HB_s$ dans tout liquide biologique humain et notamment le sérum ou le plasma, éventuellement convenablement dilué. La procédure est celle classiquement employée où après qu'a eu lieu la réaction antigène/ anticorps, on compare le signal néphélémétrique donné par la cuvette contenant l'échantillon inconnu à une courbe d'étalonnage réalisée en mesurant les signaux obtenus en présence de quantités connues de l'antigène étalon.

Toutes les possibilités techniques présentées par les néphélémètres à laser modernes telles que soustraction automatique de blanc, possibilités d'effectuer les mesures en cinétique ou en point final, automatisation miniaturisation, calculateurs incorporés traitement électronique du signal, etc. peuvent naturellement être exploitées afin d'obtenir des résultats de la meilleure qualité possible dans l'application particulière qui fait l'objet de cette invention. En outre et compte tenu de ce que les appareils de différentes marques ne présentent pas des caractéristiques et possiblités identiques il est possible de tirer parti de la très grande souplesse qu'offre le procédé objet de la présente invention pour optimiser la préparation des réactifs et les adapter à chaque type d'appareillage afin d'assurer les performances maximales.

Les exemples suivants permettent de mieux comprendre l'invention sans en limiter la portée.

**Exemple 1**

Préparation de microsphères de diamètres moyens 50 ou 110 ou 190 nanomètres:

Tous les réactifs utilisés sont d'origine commerciale et soigneusement redistillés juste avant l'emploi. La procédure générale employée est la suivante : des ampoules à sceller en verre borosilicaté de diamètre 35 mm et de capacité totale 150 ml sont préparées et l'on introduit dans chacune d'elles les quantités voulues de chacun des monomères de l'agent réticulant de l'agent tensioactif et d'eau préalablement dégazée sous vide pour obtenir dans dans tous les cas 100 ml de milieux ayant les compositions respectives données dans le tableau 1. Après barbotage d'un courant d'azote, les milieux réactionnels sont congelés par immersion dans l'azote liquide et les ampoules sont aussitôt scellées sous vide. La polymérisation est provoquée par irradiation par les rayons γ d'une bombe à $^{60}Co$. Les flux de radiations et les temps d'irradiation sont aussi donnés dans le tableau 1. Pendant toute l'irradiation les ampoules sont fixées sur un agitateur à plateau tournant à 20

tours/minute et disposées de façon à assurer la plus grande homogénéité d'irradiation. Des dosimètres disposés dans la chambre d'irradiation permettent de contrôler les doses de radiation. Après polymérisation, les ampoules sont ouvertes et leur contenu versé dans un égal volume d'une solution aqueuse à 1 g/l d'hydroquinone. Les préparations sont simplement conservées dans ces conditions en récipient bien bouché et à 4°C jusqu'à utilisation. Le diamètre des microsphères est déterminé sur des préparations examinées au microscope électronique.

**Tableau 1**

| Taille des microsphères (nm) | 50 | 110 | 190 |
|---|---|---|---|
| | Composition des milieux réactionnels (% en poids du total des monomères) | | |
| Acroléine | 45,1 | 47,0 | 47,0 |
| Méthacylate d'hydroxyéthyle | 51,3 | 49,7 | 50,7 |
| Acide méthacrylique | 2,3 | 2,0 | 1,0 |
| N-N'-méthylène bis acrylamide | 1,3 | 1,3 | 1,3 |
| Total des monomères (% du volume total du milieu) | 7,4 | 8,0 | 12,0 |
| Dodécylsulfate de sodium (g/l de milieu final) | 0,60 | 0,60 | 0,90 |
| Flux de radiation ($krad.ml^{-1}.min^{-1}$) | 0, 9 | 0,3 | 1,1 |
| Temps d'irradiation (heure) | 1,5 | 1,5 | 1,5 |

**Exemple 2**

Couplage des anticorps monoclonaux anti-$HB_s$ sur les microsphères de diamètre 50 nm:

a) Anticorps monoclonaux:

La préparation d'anticorps monoclonaux utilisée dans cette expérience est constituée par le mélange des immunoglobulines de souris monoclonales anti-$HB_s$ (de classe G et de sousclasses 1, 2a et 2b), purifiées sur colone de protéine A de Staphylocoque immobilisée sur Sépharose à partir des liquides d'ascite de souris chez qui avaient été respectivement implantés (dans le péritoine) cinq hybridomes différents producteurs d'anticorps monoclonaux spécifiques de l'antigène $HB_sa$.

b) Couplage:

La suspension de microsphères de diamètre 50 nm préparée comme indiqué dans l'exemple 1 est tout d'abord amenée à isotonicité par addition de chlorure de sodium à concentration finale 140 mM puis dialysée exhaustivement contre un tampon isotonique (PBS) constitué par du tampon phosphate 10 mM contenant du

chlorure de sodium 140 mM et ajusté à pH 7,2. Ce tampon est soigneusement dégazé avant utilisation. Une aliquote de la suspension obtenue est encore dialysée contre de l'eau pure afin de déterminer par poids sec la concentration de la suspension en microsphères.

Pour le couplage, une aliquote de suspension de microsphères dans le tampon PBS contenant 11 mg de particules, est ajoutée à une aliquote de solution d'anticorps anti-$HB_s$ contenant 10 moles d'immunoglobulines dans le même tampon et le volume total est porté à 1 ml par le tampon PBS. Après 18 heures à température ambiante sous agitation très douce on ajoute 200 microlitres d'une solution 0,2 M d'éthanolamine ajustée à pH 7,2 à l'aide d'acide chlorhydrique et l'on maintient encore 2 heures dans les mêmes conditions.

Le milieu réactionnel est déposé dans un tube à centrifuger contenant un gradient de saccharose de 10 à 60 % (poids par volume) préformé et le tube est centrifugé 2 heures à 4°C et à 20 000 tours/minute. La couche contenant les microsphères est prélevée et celles-ci sont dialysées contre 3 fois 1 litre de tampon PBS.

**Exemple 3**

Utilisation des immunosphères anti-$HB_s$ pour le dosage néphélémétrique de l'antigène $HB_s$.

a) Appareillage utilisé:

Pour les expériences décrites l'appareillage utilisé est le néphélémètre à laser PDQ HYLAND équipé d'un laser He/Ne de 0,5 mW à 632,8 nm et mesurant l'intensité lumineuse à un angle de lecture de 31,8° par rapport à l'axe du faisceau incident. Les cuvettes permettent de travailler sur un volume total de 1 ml.

b) Conditions générales des essais:

Dans tous les cas le volume total de milieu dans la cuvette est de 1 ml. Les immunosphères obtenues comme indiqué à l'exemple 2 sont introduites par 100 microlitres d'une dilution convenable de la suspension-mère qui sont additionnés en dernier au mélange préparé dans la cuvette et contenant l'échantillon à examiner dilué convenablement dans du tampon PBS contenant 1 g/l de sérum albumine bovine et 1 g/l de Tween 80 et amené à un volume total de 900 microlitres par le même diluant. La réaction d'agglutination spécifique génératrice du signal néphélémétrique débute lors de l'addition des immunosphères et peut être suivie dans le temps (lecture cinétique) ou mesurée à temps fixe. Sauf indication contraire la mesure est effectuée au temps 1 heure après mise en contact des immunosphères spécifiques avec l'antigène dans la cuvette. L'expérience a montré qu'après ce délai l'essentiel du signal néphélémétrique spécifique est obtenu et que la prolongation du délai jusqu'à 8 ou 16 heures n'apporte pas d'avantage pratique notable. Tous les résultats présentés ci-après sont donnés après soustraction des blancs correspondant à chaque essai et représentent donc le signal spécifique de la réaction évaluée.

c) Echantillons utilisés:

- un sérum humain de référence titré à 31 microgrammes d'antigène $HB_s$ par millilitre,
- un sérum humain positif d'origine hospitalière titrant environ 5 microgrammes par ml.

d) Etudes réalisées:

1° Stabilité des suspensions d'immunosphères:

Les signaux correspondant à quatre suspensions différentes d'immunosphères anti-$HB_s$ seules (sans antigène) de concentrations comprises entre 3,5 et 28 µg/ml ont été mesurés répétitivement pendant une période de 96 heures sans agitation du contenu des cuvettes. Ces signaux se révèlent d'une excellente stabilité puisque les valeurs extrêmes observées ne s'écartent jamais de plus de 2 % de la valeur moyenne pour la suspension la plus concentrée et de plus de 7 % pour la suspension la plus diluée. Ce résultat démontre l'extrême stabilité des suspensions d'immunosphères dans les conditions pratiques de leur utilisation, y compris pour des délais incomparablement plus longs que ceux qui sont nécessaires aux applications analytiques.

2° Etalonnage du dosage:

2-1- Lorsque la concentration des immunosphères est fixée à 7 microgrammes par millilitre, une gamme d'étalonnage effectuée à partir de dilutions du sérum étalon montre que le signal néphélémétrique spécifique permet d'explorer valablement un domaine de concentrations en antigène $HB_s$ extrêmement vaste puisqu'il s'étend depuis environ 0,15 ng/ml jusqu'à près de 80 ng/ml. Cette courbe d'étalonnage est représentée sur la figure 1 sur laquelle on a porté en ordonnée l'intensité lumineuse diffusée (en unités arbitraires) et en abscisse la concentration antigène $HB_s$ en nanogrammes par millilitre.

Cette caractéristique présente un grand intérêt pratique dans la mesure où elle évite à l'utilisateur de devoir préparer un grand nombre de dilutions d'un échantillon inconnu pour obtenir une mesure comprise dans la partie exploitable de la gamme d'étalonnage.

2-2- Lorsque la concentration des immunosphères est abaissée jusqu'à 1,4 microgramme par millilitre, une courbe d'étalonnage analogue montre que la sensibilité limite peut aisément être abaissée jusqu'à une concentration d'antigène de l'ordre ou même inférieure à 0,1 ng/ml. Cette courbe d'étalonnage est représentée sur la figure 2 sur laquelle on a porté en ordonnée l'intensité lumineuse diffusée (en unités arbitraires) et en abscisse la concentration antigène HB en nanogramme par millilitre. Ce résultat est extrêmement important puisqu'il démontre que la méthode mise au point permet d'atteindre des sensibilités limites au moins aussi élevées que celles des meilleurs dosages radio-immunologiques actuellement disponibles sans présenter aucun des inconvénients liés à la manipulation de substances radioactives.

3° Contrôles de spécificité :

Si, dans des conditions identiques à celles du paragraphe 2-1, on incube les solutions d'antigène pendant 24 heures avec de l'anticorps libre (non couplé aux immunosphères) à la concentration finale de 70 microgrammes/millilitre, aucun signal distingable du blanc de réactifs n'apparaît. Ceci démontre qu'en l'absence de son couplage aux immunosphères l'anticorps spécifique, même à concentration élevée, est incapable de provoquer l'apparition d'un signal néphélémétrique spécifique.

Si dans les mêmes cuvettes, on ajoute alors les immunosphères spécifiques, on n'observe à nouveau aucune apparition d'un signal spécifique. Ceci démontre que l'antigène présent a bien été neutralisé par l'excès d'anticorps libre dans la première étape et n'est donc plus disponible pour provoquer l'agglutination spécifique des immunosphères dans la deuxième étape. Ce résultat démontre la spécificité de reconnaissance de l'antigène $HB_s$ par les immunosphères utilisées.

4° Dosage d'un sérum inconnu :

4-1 Afin de permettre une évaluation réaliste de la capacité des réactifs objets de la présente invention à assurer une grande souplesse d'utilisation dans le dosage de l'antigène $HB_s$ dans des sérums de titre inconnu, un sérum humain de provenance hospitalière a été employé. Une série de 14 dilutions de ce sérum en progression géométrique de raison 2 a été réalisée et des aliquotes de toutes ces dilutions ont été soumises à la réaction avec les immunosphères dans les conditions décrites au paragraphe 2-1. Les courbes de la figure 3 montrent que le signal néphélémétrique spécifique de toutes ces dilutions est aisément mesurable à condition de choisir la gamme de sensibilité convenable de l'appareil et jusqu'à des dilutions extrêmement grandes de sérum de départ puisque le signal est encore significatif pour une dilution au 1/100 000. Cette observation démontre qu'en pratique avec un maximum de 5 dilutions on peut en une seule étape dont la durée ne dépasse pas une heure obtenir le dosage d'un sérum inconnu quel que soit son titre entre 1 ng/ml et 10 µg/ml.

Sur cette figure n°3, on a porté, en ordonnée, l'intensité lumineuse en unités arbitraires et, en abscisse la dilution du sérum: Les mesures ont été effectuées avec des immunosphères de 7 mg/ml et avec trois sensibilités différentes de l'appareil.

4-2- Dans le cas précis du sérum utilisé la comparaison des signaux obtenus pour les dilutions les mieux situées dans la gamme de mesure avec la courbe d'étalonnage de la figure 1 conduit à une valeur moyenne du titre du sérum de 5,9 mg d'antigène $HB_s$ par ml , les valeurs extrêmes notées sur 6 mesures en simple exemplaire à des dilutions différentes étant 5,3 et 6,4. Cette précision est excellente pour des dosages de ce type.

5° Répétabilité des résultats:

Dans des conditions analogues à celles indiquées ci-dessus lorsque pour deux dilutions différentes de sérum et sur les gammes de sensibilité correspondantes on mesure les signaux donnés par 10 cuvettes indépendantes mais de composition identique, l'analyse statistique des valeurs obtenues conduit aux résultats suivants:

| | 1ère dilution (1/40) | 2e dilution (1/5120) |
|---|---|---|
| Nombre de mesures | 10 | 10 |
| Signal moyen (unités arbitraires) | 52,2 | 69,5 |
| Ecart-type | 2,72 | 4,36 |
| Coefficient de variation (%) | 5,2 | 6,3 |

Ces résultats confirment l'excellente répétabilité de l'ensemble du dosage y compris en particulier la réaction d'agglutination des immunosphères spécifiques par l'antigène à doser.

Ces différents essais mettent en évidence les caractéristiques et avantages de la méthode de dosage de l'antigène $HB_s$ à l'aide des réactifs et procédés objets de la présente invention. Ils démontrent en particulier :

- la très grande sensibilité du dosage qui atteint des seuils de détection au moins aussi bas que ceux des meilleures méthodes actuellement disponibles pour ce même dosage (de l'ordre de 0,1 ng d'antigène par ml);
- la haute spécificité assurée par le choix d'anticorps monoclonaux strictement spécifiques de l'antigène à doser;

- la facilité de mise en oeuvre de l'ensemble du dosage dont les opérations se limitent à quelques pipettages en microméthode qui peuvent indifféremment être réalisés soit manuellement soit d'une façon entièrement automatisée;

- la très bonne conservation des réactifs qui restent valables pendant des mois (et problablement des années) à 4° C;

- le faible prix de revient de chaque dosage qui n'exige pas de personnel de haute qualification et qui ne consomme que des quantités extrêmement faibles de réactifs (de quelques microgrammes à quelques dizaines de microgrammes d'immunosphères par essai, selon les appareils utilisés);

- la possibilité d'adapter la technique à tous les néphélémètres à laser commerciaux qui sont communément répandus dans les laboratoires, sans perte importante de performances globales;

- le court délai de réponse de l'ensemble de la manipulation analytique puisque l'étape la plus longue est celle requise par la réaction entre antigène et anticorps et que celle-ci peut ne pas dépasser une heure;

- la versatilité de la méthode: en effet les exemples décrits concernent seulement le dosage direct de l'antigène par agglutination des immunosphères portant l'anticorps spécifique. Selon un principe tout à fait analogue, on peut aussi bien coupler sur les microspères l'antigène purifié. Si l'on introduit alors dans les cuvettes de mesure des microsphères à antigène en quantité constante et de l'anticorps spécifique également en quantité constante, pour assurer l'agglutination spécifique génératrice du signal néphélémétrique, on dispose d'un système dans lequel toute introduction d'antigène libre sous la forme d'une aliquote convenablement diluée d'un sérum à doser ( ou d'un sérum étalon) provoquera une inhibition mesurable du signal de base d'où la possibilité de réaliser un dosage quantitatif par inhibition.

Les coffrets de réactifs qui seront mis sur le marché en application de la présente invention comprendront notamment

a) pour la mesure directe de l'antigène :

Le réactif spécifique sous la forme d'immunosphères porteuses de l'anticorps spécifique anti-$HB_s$ qu'il soit polyclonal ou constitué d'une ou plusieurs immunoglobulines monoclonales entières ou sous forme de fragments ayant conservé la capacité de reconnaissance de l'antigène. Ce réactif sera présenté soit en solution à diluer ou non pour l'emploi, soit lyophilisé à reconstituer pour l'emploi dans un solvant indiqué à l'utilisateur ou livré dans le coffret, toutes ces préparations pouvant contenir en outre des additifs tels que conservateurs et stabilisants.

b) pour la mesure de l'antigène par inhibition :

Deux réactifs spécifiques qui sont:

- des microsphères porteuses de l'antigène spécifique, présentées dans des conditions analogues à celles décrites pour les immunosphères spécifiques du cas a) ci-dessus,

- un agent capable de provoquer l'agglutination tel que toute préparation convenable contenant un antisérum total anti-$HB_s$, ou des immunoglobulines spécifiques polyclonales ou monoclonales anti-$HB_s$, purifiées ou non, entières ou à l'état de fragments comportant la capacité de reconnaissance de l'antigène spécifique et d'agglutination des microsphères portant l'antigène spécifique. Cet agent sera présenté soit en solution tamponnée ou non, à diluer avant emploi ou non soit sous forme de lyophilisats à reconstituer dans un solvant indiqué à l'utilisateur ou livré dans le coffret, cette préparation pouvant contenir en outre des additifs tels que conservateurs et stabilisants.

En outre, quel que soit le mode de dosage adopté, le coffret pourra comprendre des réactifs communs qui seront notamment :

- un diluant
- une préparation étalon d'antigène $HB_s$
- des préparations de contrôle telles que par exemple des sérums de contrôle de concentration basse, moyenne et haute par rapport à l'étendue de la gamme normale de dosage
- un sérum de contrôle négatif, garanti exempt d'antigène $HB_s$.

Ces réactifs communs seront présentés soit en solution tamponnée ou non, à diluer avant l'emploi ou non soit sous forme de lyophisats à reconstituer dans un solvant indiqué à l'utilisateur ou livré dans le coffret. Ces préparations pourront en outre contenir des additifs tels que conservateurs, stabilisants, agents tensio-actifs, agents de dispersion, tous ces additifs pouvant être macromoléculaires (et en particulier protéiques) ou non.

**Revendications**

1. Microparticules sphériques hydrophiles, polyfonctionnelles de taille uniforme, de diamètres moyens entre environ 10 nanomètres et environ 10 micromètres, caractérisées en ce qu'elles sont obtenues par copolymérisation des monomères ci-après:

1. au moins 5 % en poids d'un aldéhyde acrylique de formule $CH_2 =$ 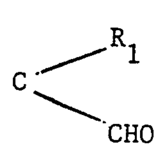

dans laquelle $R_1$ est l'hydrogène ou un radical alkyle inférieur ayant de 1 à 4 atomes de carbone;

2. au moins 5 % en poids d'un dérivé acrylique de formule $CH_2$ = 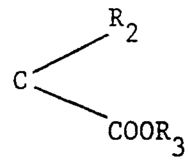

dans laquelle $R_2$ est choisi parmi l'hydrogène et les groupes alkyle inférieur ayant de 1 à 4 atomes de carbone et $R_3$ est un radical alkyle inférieur hydroxylé ;

3. de moins de 15 % en poids d'au moins un dérivé acrylique choisi parmi les dérivés de formule

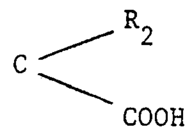

dans laquelle $R_2$ est tel que défini ci-dessus, et 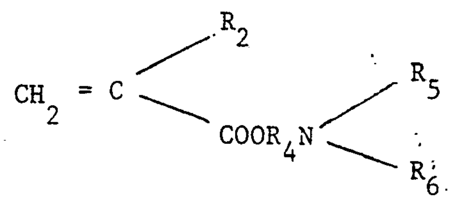

dans laquelle $R_4$, $R_5$, $R_6$ sont des groupes alkyle inférieur (1 à 4 atomes de carbone) identiques ou différents et $R_2$ est tel que défini ci-dessus, en présence de 0,1 à 10 % en poids d'un diène non conjugué comme agent réticulant et d'un agent tensio-actif, les proportions en monomères et en agent réticulant sont exprimées en poids de l'ensemble des monomères.

2. Microparticules sphériques hydrophiles selon la revendication 1, caractérisées en ce que la proportion du monomère aldhéhyde acrylique est comprise entre 40 et 60 % en poids.

3. Microparticules sphériques hydrophiles selon l'une des revendications 1 ou 2, caractérisées en ce que la proportion du monomère 2) est comprise entre 40 et 70 % en poids.

4. Procédé pour la préparation de microparticules sphériques hydrophiles, polyfonctionnelles de taille uniforme, de diamètres moyens entre environ 10 nanomètres et environ 10 micromètres, caractérisé en ce que l'on copolymérise en milieu aqueux les monomères ciaprès dans les porportions suivantes:

1. au moins 5 % en poids d'un aldéhyde acrylique de formule 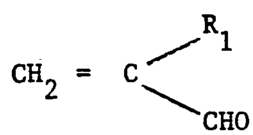

dans laquelle $R_1$ est l'hydrogène ou un radical alkyle inférieur ayant de 1 à 4 atomes de carbone;

2. au moins 5 % en poids d'un dérivé acrylique de formule 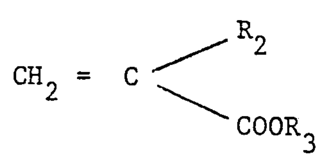

dans laquelle $R_2$ est choisi parmi l'hydrogène et les groupes alkyle inférieur ayant de 1 à 4 atomes de carbone et $R_3$ est un radical alkyle inférieur hydroxylé;

3. de moins de 15 % en poids d'au moins un dérivé acrylique choisi parmi les dérivés de formule

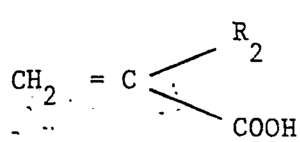

dans laquelle $R_2$ est tel que défini ci-dessus et les dérivés de formule:

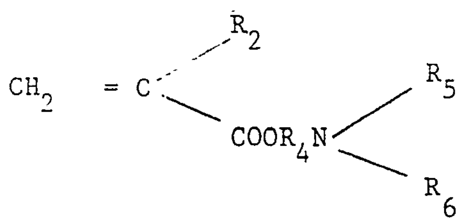

dans laquelle $R_4$, $R_5$, $R_6$ sont des groupes alkyle inférieurs (1 à 4 atomes de carbone) identiques ou différents et $R_2$ est tel que défini ci-dessus, ladite copolymérisation étant réalisée en présence de 0,1 à 10 % en poids d'un diène non conjugué comme agent réticulant et d'un agent tensio-actif soluble dans l'eau, les proportions sont en poids de l'ensemble des monomères.

5. Procédé selon la revendication 4 caractérisé en ce que ledit agent tensioactif est de nature ionique ou non ionique et qu'il est utilise a raison de 0,01 à 5 % en poids du milieu réactionnel total.

6. Procédé selon l'une des revendications 4 ou 5, caractérisé en ce que la concentration totale des monomères dans le milieu aqueux de polymérisation est compris entre 4 et 16 % en poids.

7. Procédé selon l'une quelconque des revendications 4 à 6, caractérisé en ce que la copolymérisation est réalisée sous l'influence d'un rayonnement de haute énergie ( tel que le rayonnement $\gamma$ du cobalt) utilisé à une dose de 0,01 à 0,5 mégarad par heure et par $cm^3$ du mélange et pendant une durée d'au moins 15 minutes, la solution de copolymérisation étant agitée.

8. Utilisation des microparticules selon la revendication 1, pour l'obtention d'immunosphères caractérisée en ce que lesdites microparticules, convenablement purifiées, sont mises en contact dans un milieu liquide avec un anticorps choisi parmi :

- les anticorps polyclonaux et isolés par les techniques connues à partir d'immu-sérums spécifiques obtenus chez l'animal après immunisation par l'antigène $HB_s$ purifie,
- et les anticorps monoclonaux et purifiés par les méthodes connues après production à partir de cellules hybrides (hybridomes) résultant de la fusion de plasmocytes d'animaux immunisés contre l'antigène $HB_s$ et de cellules de myélome possédant les caractéristiques convenables.

9. Utilisation des microparticules selon la revendication 8, caractérisée en ce qu'après mise en contact desdites particules avec ledit anticorps le produit obtenu est traité avec une amine primaire en excès de façon à bloquer les fonctions aldéhydes libres.

10. Utilisation des microparticules selon la revendication 8, caractérisée en ce qu'après mise en contact desdites particules avec ledit anticorps le produit obtenu est réduit par traitement à l'aide d'un hydrure métallique.

11. Utilisation des immunosphères selon l'une quelconque des revendications 7 à 10 pour le dosage néphélémétrique de l'antigène $HB_s$ dans un liquide biologique.

**Patentansprüche**

1. Polyfunktionelle, hydrophile, kugelförmige Mikroteilchen von einheitlicher Größe mit mittleren Durchmessern zwischen etwa 10 Nanometern und etwa 10 Mikrometern, dadurch gekennzeichnet, daß sie erhalten sind durch Copolymerisation der nachstehenden Monomere:

1. mindestens 5 Gew. % eines Acrylaldehyds der

Formel 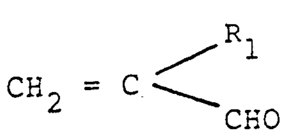 ,

worin $R_1$ Wasserstoff oder eine Niedrigalkylgruppe mit 1 bis 4 Kohlenstoffatomen ist;

2. mindestens 5 Gew. % eines Acrylderivats der Formel 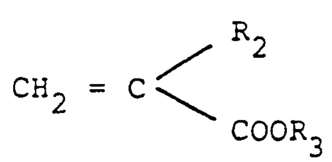 ,

worin $R_2$ ausgewählt ist aus Wasserstoff und den Niedrigalkylgruppen mit 1 bis 4 Kohlenstoffatomen und $R_3$ eine hydroxylierte Niedrigalkylgruppe ist;

3. mindestens 15 Gew. % mindestens eines Acrylderivats ausgewählt aus den Derivaten der Formel

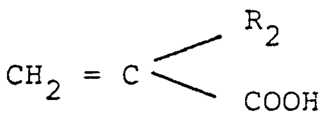 , worin $R_2$ obige Bedeutung hat, und

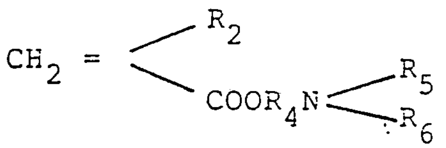 ,

.

worin $R_4$, $R_5$, $R_6$ gleiche oder verschiedene Niedrigalkylgruppen (1 bis 4 Kohlenstoffatome) sind und $R_2$ obige Bedeutung hat, in Gegenwart von 0,1 bis 10 Gew. % eines nichtkonjugierten Diens als Vernetzungsmittel und eines grenzflächenaktiven Mittels, wobei die Anteile an Monomeren und an Vernetzungsmittel in Gew. % der Gesamtheit der Monomere ausgedrückt sind.

2. Hydrophile kugelförmige Mikroteilchen nach Anspruch 1, dadurch gekennzeichnet, daß der Anteil des Acrylaldehydmonomeren zwischen 40 und 60 Gew. % beträgt.

3. Hydrophile kugelförmige Mikroteilchen nach einem der Ansprüche 1 oder 2, dadurch gekennzeichnet, daß der Anteil des Monomeren 2) zwischen 40 und 70 Gew. % beträgt.

4. Verfahren zur Herstellung von polyfunktionellen, hydrophilen, kugelförmigen Mikroteilchen von einheitlicher Größe mit mittleren Durchmessern zwischen etwa 10 Nanometern und etwa 10 Mikrometern, dadurch gekennzeichnet, daß man die nachstehenden Monomere in folgenden Anteilen in wässerigem Milieu copolymerisiert:

1. mindestens 5 Gew. % eines Acrylaldehyds der Formel 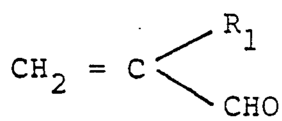 ,

worin $R_1$ Wasserstoff oder eine Niedrigalkylgruppe mit 1 bis 4 Kohlenstoffatomen ist;

2. mindestens 5 Gew. % eines Acrylderivats der Formel 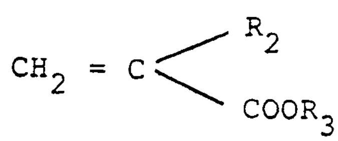 ,

worin $R_2$ ausgewählt ist aus Wasserstoff und den Niedrigalkylgruppen mit 1 bis 4 Kohlenstoffatomen und $R_3$ eine hydroxylierte Niedrigalkylgruppe ist;

3. mindestens 15 Gew. % mindestens eines Acrylderivats ausgewählt aus den Derivaten der Formel

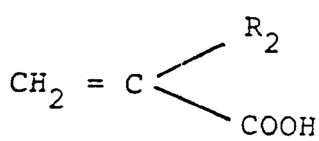 ,

worin $R_2$ obige Bedeutung hat, und den Derivaten der Formel

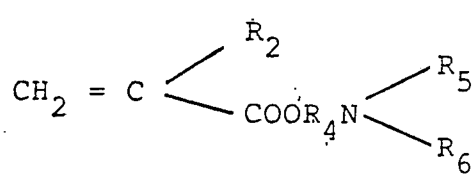 ,

worin $R_4$, $R_5$, $R_6$ gleiche oder verschiedene Niedrigalkylgruppen (1 bis 4 Kohlenstoffatome) sind und $R_2$ obige Bedeutung hat, welche Copolymerisation in Gegenwart von 0,1 bis 10 Gew. % eines nichtkonjugierten Diens als Vernetzungsmittel und eines wasserlöslichen grenzflächenaktiven Mittels, wobei die Anteile in Gew. % der Gesamtheit der Monomere ausgedrückt sind, durchgeführt wird.

5. Verfahren nach Anspruch 4, dadurch gekennzeichnet, daß das grenzflächenaktive Mittel ionischer oder nichtionischer Natur ist und in einer Menge von 0,01 bis 5 Gew. % des gesamten Reaktionsmilieus verwendet wird.

6. Verfahren nach einem der Ansprüche 4 oder 5, dadurch gekennzeichnet, daß die Gesamtkonzentration an Monomeren im wasserigen Polymerisationsmilieu zwischen 4 und 16 Gew. % beträgt.

7. Verfahren nach einem der Ansprüche 4 bis 6, dadurch gekennzeichnet, daß die Copolymerisation unter Einwirkung einer hochenergetischen Strahlung (wie Kobalt-γ-Strahlung) in einer Dosis von 0,01 bis 0,5

Megarad pro Stunde und pro $cm^3$ Mischung und während eines Zeitraums von mindestens 15 min durchgeführt wird, wobei die Copolymerisationslösung gerührt wird.

8. Verwendung der Mikroteilchen nach Anspruch 1 zur Schaffung von Immunkügelchen, dadurch gekennzeichnet, daß die Mikroteilchen entsprechend gereinigt in einem flüssigen Milieu in Kontakt mit einem Antikörper gebracht werden, der ausgewählt ist aus:

- den polyklonalen und durch bekannte Methoden aus beim Tier nach Immunisierung mit dem gereinigten $HB_s$-Antigen erhaltenen spezifischen Immunseren isolierten Antikörpern,
- und den monoklonalen und durch bekannte Methoden nach der Herstellung aus Hybridzellen (Hybridomen), die aus der Verschmelzung von gegen das $HB_s$-Antigen immunisierten Tierplasmozyten und von die geeigneten Merkmale aufweisenden Myelomzellen resultieren, gereinigten Antikörpern.

9. Verwendung der Mikroteilchen nach Anspruch 8, dadurch gekennzeichnet, daß nach in Kontaktbringen der Teilchen mit dem Antikörper das erhaltene Produkt mit einem primären Amin im Überschuß derart behandelt wird, daß die freien Aldehydfunktionen blockiert werden.

10. Verwendung der Mikroteilchen nach Anspruch 8, dadurch gekennzeichnet, daß nach in Kontaktbringen der Teilchen mit dem Antikörper das erhaltene Produkt durch Behandlung mit einem Metallhydrid reduziert wird.

11. Verwendung der Immunkügelchen nach einem der Ansprüche 7 bis 10 zur nephelometrischen Bestimmung des $HB_s$-Antigens in einer biologischen Flüssigkeit.

**Claims**

1. Polyfunctional hydrophilic spherical microparticles of uniform size, having average diameters between about 10 nanometers and about 10 micrometers, characterized in that they are obtained by copolymerizing the following monomers:

1. at least 5% by weight of an acrylic aldehyde of formula 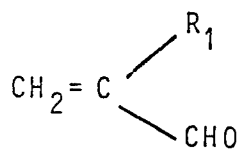

in which $R_1$ is hydrogen or a lower alkyl radical having 1 to 4 atoms of carbon;

2. at least 5% by weight of an acrylic derivative of formula 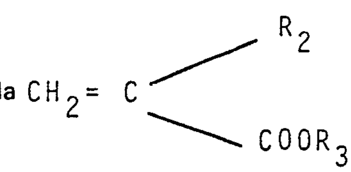

in which $R_2$ is selected from hydrogen and the lower alkyl groups with 1 to 4 atoms of carbon and $R_3$ is a hydroxylated lower alkyl radical;

3. Less than 15% by weight of at least an acrylic derivative selected from the derivatives of formula

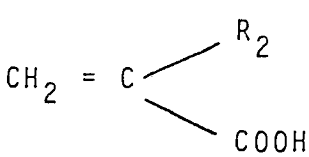 in which $R_2$ is such as defined above, and

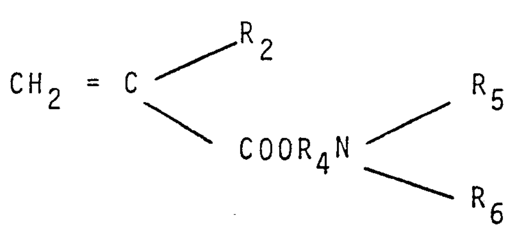

in which $R_4$, $R_5$, $R_6$ are identical or different lower alkyl groups (1 to 4 atoms of carbon) and $R_2$ is such as defined above, in the presence of 0.1 to 10% by weight of a nonconjugated diene as cross-linking agent and of a surface-active agent, the proportions of monomers and of cross-linking agent being expressed by weight of all the monomers.

2. Hydrophilic spherical microparticles according to claim 1, characterized in that the proportion of the acrylic aldehyde monomer is between 40 and 60% by weight.

3. Hydrophilic spherical microparticles according to any one of claims 1 or 2, characterized in that the proportion of monomer 2) is between 40 and 70% by weight.

4. Process for preparing polyfunctional hydrophilic spherical microparticles, of uniform size, with average diameters between about 10 nanometers and about 10 micrometers, characterized in that the following monomers are copolymerized in aqueous medium in the following proportions :

1. at least 5% by weight of an acrylic aldehyde of formula 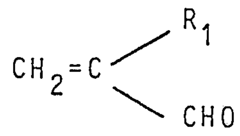

in which $R_1$ is hydrogen or a lower alkyl radical having 1 to 4 atoms of carbon;

2. at least 5% by weight of an acrylic derivative of formula 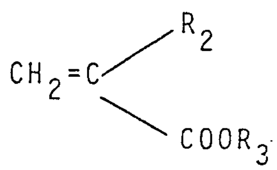

in which $R_2$ is selected from hydrogen and the lower alkyl groups having from 1 to 4 atoms of carbon and $R_3$ is a hydroxylated lower alkyl radical;

3. less than 15% by weight of at least an acrylic derivative selected the derivatives of formula

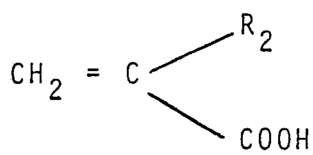

in which $R_2$ is such as defined above and the derivatives of formula: 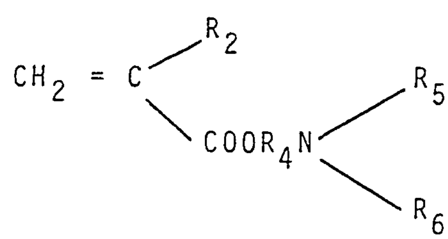

in which $R_4$, $R_5$, $R_6$ are identical or different lower alkyl groups (1 to 4 atoms of carbon) and $R_2$ is such as defined above, said copolymerization being carried out in the presence of 0.1 to 10% by weight of a nonconjugated diene as cross-linking agent and of a surface-active agent soluble in water, the proportions are expressed by weight of all the monomers.

5. Process according to claim 4, characterized in that said surface-active agent is of ionic or non ionic nature and in that it is used at the rate of 0.01 to 5% by weight of the total reaction medium.

6. Process according to any one of claims 4 or 5, characterized in that the total concentration of monomers in the polymerizing aqueous medium is between 4 and 16% by weight.

7. Process according to any one of claims 4 to 6, characterized in that the copolymerization is carried out under the influence of a high-energy radiation (such as the $\gamma$ radiation of cobalt) used in the proportion of 0.01 to 0.5 megarad per hour and per $cm^3$ of the mixture,and for a duration of at least 15 minutes, the copolymerizing solution being stirred.

8. Use of microparticles according to claim 1 for obtaining immunospheres, characterized in that said appropriately purified microparticles are placed in contact in a liquid medium with an antibody selected from:

- the polyclonal antibodies isolated by known techniques from specific immune sera obtained from the animal after immunization by purified $HB_s$ antigen,

- and the monoclonal antibodies purified by the known methods after production from hybrid-cells (hybridomas) resulting from the fusing plasmocytes of animals immunized against $Hb_s$ antigen with myeloma cells having appropriate characteristics.

9. Use of microparticles according to claim 8, characterized in that after said particles are placed in contact with said antibody, the resulting product is treated with an excess of primary amine so as to block the free aldehyde functions.

10. Use of microparticles according to claim 8, characterized in that after said particles are placed in contact with said antibody the product obtained is reduced by treatment with a metallic hydride.

11. Use of immunospheres according to any one of claims 7 to 10 for the nephelometric determination of the $HB_s$ antigen in a biological liquid.

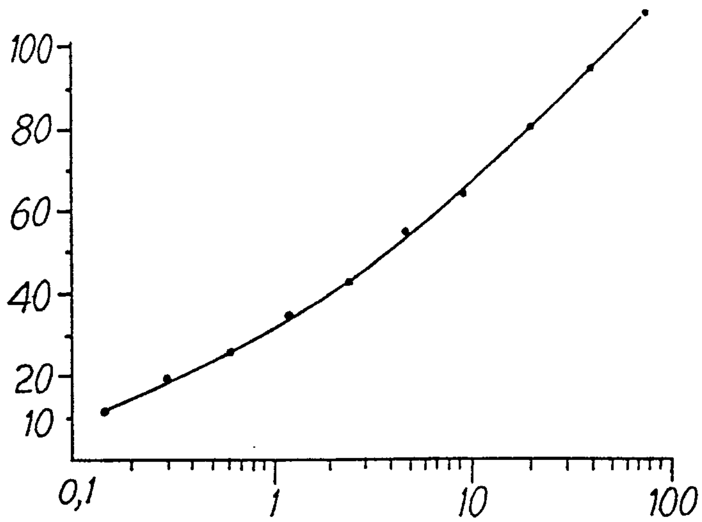


Fig.1

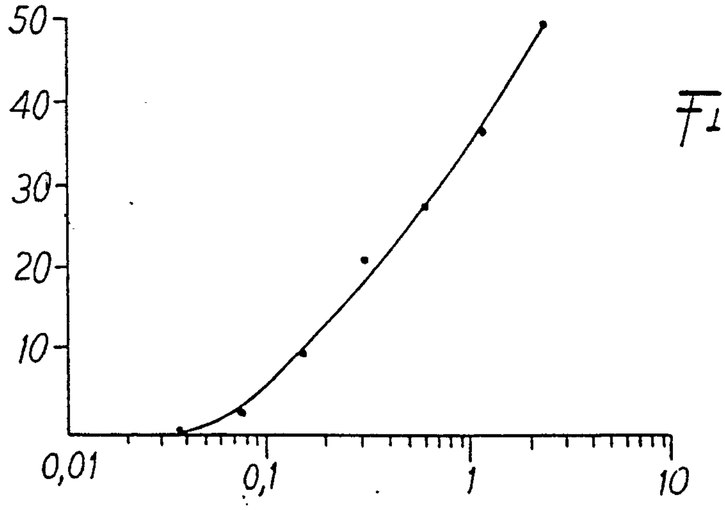


Fig.2

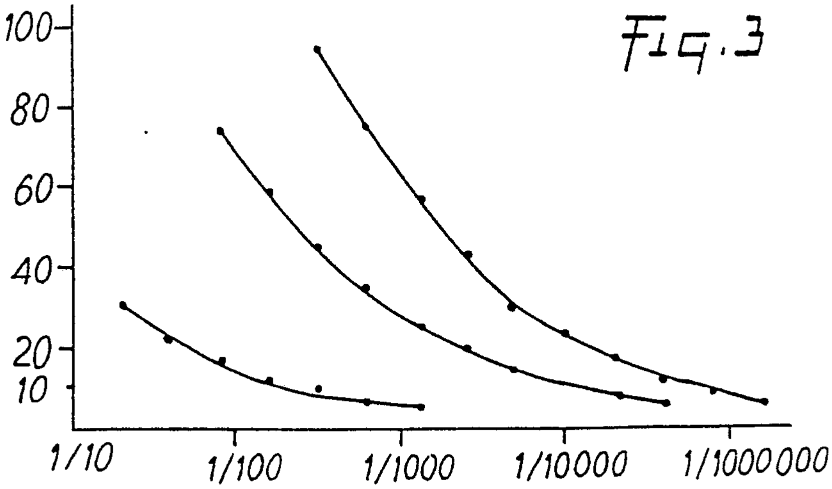


Fig.3